# DEMANDE DE BREVET EUROPEEN

(11) **EP 1 491 495 A1**
(43) Date de publication de la demande: **29.12.2004**
(21) Numéro de dépôt: 04447145.6
(22) Date de dépôt: 21.06.2004
(51) Int. Cl.: C01B 13/10, C01B 13/11, A61L 2/18

(54) **Générateur et microdoseur électronique d'ozone dans l'eau**

(30) Priorité: 26.06.2003 US 483259 P
(71) Demandeur: Patti, Antonino, 4342 Hognoul (BE)
(72) Inventeur: Patti, Antonino, 4342 Hognoul (BE)
(74) Mandataire: Van Malderen, Michel

(57) **Abrégé**

La présente invention se rapporte à un appareil électronique doseur d'ozone dissous dans l'eau, comprenant au moins un tube générateur (14) de l'ozone à partir d'oxygène diatomique pur ou présent dans un gaz et un venturi (3) connecté d'une part audit générateur (14) et d'autre part à une alimentation en eau (1), ledit venturi servant au mélange de l'ozone produit avec l'eau, ainsi qu'un boîtier électrique (10) contenant un transformateur (13) pour l'alimentation haute tension (HT) du tube générateur et une carte électronique de commande et de contrôle à microcontrôleur (12), caractérisé en ce que ledit appareil comprend des moyens de sécurité pour détecter un courant de fuite au niveau de la haute tension d'alimentation du tube générateur, notamment en cas de remontée d'eau ou de présence d'humidité au niveau du tube générateur.

## Description

### Objet de l'invention

La présente invention se rapporte à un générateur-doseur d'ozone dissous dans l'eau, commandé par des moyens électroniques.

Un domaine d'application est l'utilisation de l'appareil de l'invention à des fins médicales, et plus particulièrement en dermatologie. L'utilisation de l'appareil permet notamment de procurer une garantie de propreté totale des mains des travailleurs de la santé.

L'action désinfectante directe de l'appareil peut être couplée à toute action de stérilisation de locaux, de mobiliers hospitaliers et d'instruments médicaux, chirurgicaux, etc.

Un autre domaine d'application de choix est la désinfection dans le domaine agro-alimentaire, le remplacement des pesticides dans le traitement des terres cultivables, etc.

### Arrière-plan technologique et état de la technique

L'effet oxydant, bactéricide, fongicide, antiviral, désinfectant, stérilisant, etc., de l'ozone est bien connu. A des fins pratiques, on essaie généralement de dissoudre l'ozone dans de l'eau qui sera utilisée ultérieurement lors d'un traitement visant par exemple à la stérilisation de l'eau ou encore la destruction de germes pathogènes.

La difficulté technique essentielle consiste généralement à trouver la dose optimale d'ozone à dissoudre dans l'eau pour éviter l'obtention d'une solution soit trop oxydante, soit inefficace lors du traitement.

Dans le cas d'espèce de l'invention, l'eau est utilisée comme vecteur pour l'ozone à des fins de traitement thérapeutique. L'intervalle de concentration généralement désirée d'ozone dissous dans l'eau se situe entre 0,03 et 0,08ppm. Au-delà de 0,08ppm de concentration d'ozone dans l'eau, on atteint un effet stérilisant qui n'est pas toujours souhaité. En dessous de 0,03ppm, l'effet de l'ozone diminue fortement et les durées de traitement augmentent considérablement. Au sein de l'intervalle de 0,03 à 0,08ppm, on atteint généralement un effet thérapeutique qui consiste à soigner des parties du corps, la peau en particulier, les concentrations utilisées étant insuffisantes pour abîmer celle-ci. Les concentrations d'ozone dans la plage précitée entraînent notamment un effet "peeling" de la peau. Il y a également un nettoyage des pores de la peau.

L'ozone peut également être utilisé dans ces conditions pour entraîner un effet phytosanitaire sur les plantations et remplacer en tout ou en partie les pesticides.

L'ozone est généralement produit par décharge électrique (effet corona) dans un générateur à tubes. Le générateur d'ozone est composé de deux électrodes conductrices formant un condensateur et soumises à une différence de potentiel de haute tension. Lorsque de l'air ou de l'oxygène éventuellement comprimé passe dans le champ électrique régnant entre ces deux électrodes, une partie des molécules d'oxygène diatomique (O₂) est transformée en molécules d'ozone triatomique (O₃).

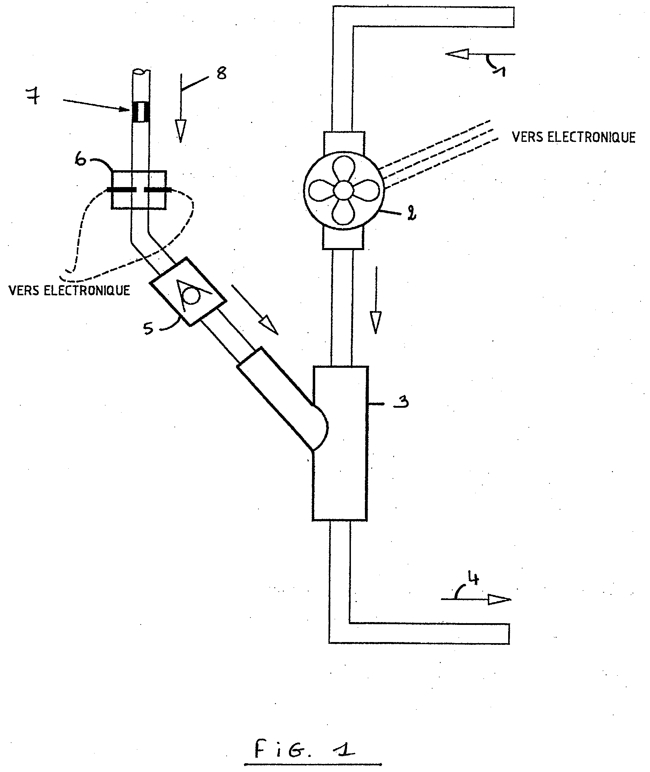

L'ozone est ensuite généralement mélangé à l'eau par utilisation d'un venturi qui permet d'introduire ce gaz dans l'eau courante, comme représenté sur la figure 1.

La difficulté essentielle consiste à réguler la quantité d'ozone dissous dans l'eau et, à cette fin, il importe de maîtriser la tension d'alimentation des tubes générateurs d'ozone.

De plus, dans la mesure où la pression d'alimentation en eau courante varie d'un endroit à l'autre, les quantités d'ozone dissous sont essentiellement conditionnées par cette variation de pression (en plus de la variation éventuelle de température bien sûr).

Un autre problème critique est celui de la sécurité d'utilisation de ce type d'installation dans des douches ou des productions d'eau qui sont directement appliquées sur le corps, la peau en particulier, et qui doivent satisfaire à des conditions de sécurité drastiques.

Dans l'état de la technique, on utilise des contacteurs différentiels disponibles dans le commerce, qui ont un niveau de sensibilité descendant jusqu'à 10 milliampères, ce qui est insuffisant.

### Buts de l'invention

La présente invention vise à fournir un appareil générateur d'ozone tel que la quantité d'ozone dissous dans l'eau est contrôlée.

En particulier, l'invention vise à fournir un appareil qui permette de maîtriser la tension d'alimentation des tubes générateurs d'ozone.

Un but complémentaire de l'invention est de sécuriser les installations productrices d'eau contenant de l'ozone dissous, telles que des douches.

Un but complémentaire de l'invention est de prévoir une implantation des composants sur la carte électronique de contrôle, respectant des distances d'isolement compatibles avec l'amenée de haute tension sur la carte.

Un but supplémentaire de l'invention est de fournir une carte sur laquelle tous les composants électroniques peuvent être soudés, à l'exception du microcontrôleur, ce qui permet d'obtenir un appareil plus compact.

### Principaux éléments caractéristiques de l'invention

La présente invention se rapporte à un appareil électronique doseur d'ozone dissous dans l'eau, comprenant au moins un tube générateur de l'ozone à partir d'oxygène diatomique pur ou présent dans un gaz (l'air par exemple) et un venturi connecté d'une part audit générateur et d'autre part à une alimentation en eau, ledit venturi servant au mélange de l'ozone produit avec l'eau, ainsi qu'un boîtier électrique contenant un transformateur pour l'alimentation haute tension (HT) du tube générateur et une carte électronique de commande et de contrôle à microcontrôleur ou à microprocesseur, caractérisé en ce que ledit appareil comprend des moyens de sécurité pour détecter un courant de fuite au niveau de la haute tension, notamment en cas de remontée d'eau ou de présence d'humidité au niveau du tube générateur.

Selon l'invention, lesdits moyens comprennent un contacteur différentiel ultrasensible permettant de court-circuiter un fusible rapide de protection de la carte électronique, ce qui coupe l'alimentation HT, par détection du passage d'un courant de fuite à partir d'un seuil d'au plus 2mA en un temps inférieur à 20ms.

Selon une forme d'exécution particulièrement préférée, le contacteur différentiel est tel que le point milieu du transformateur HT est connecté à un réseau de résistances limitatives de tension et est relié à la terre via un optocoupleur à sortie Darlington, qui commande, via un transistor, un relais mettant en court-circuit ledit fusible rapide de protection.

Un avantage majeur de la présente invention est que le contacteur différentiel est intégré à la carte électronique. Par ailleurs, la haute tension du transformateur d'alimentation du tube générateur d'ozone est amenée sur la carte au moyen de colonnettes hexagonales fixées à la carte et de hauteur minimale 20mm, ce qui permet de respecter la distance d'isolement nécessaire.

De manière également avantageuse, le relais est commandé par deux transistors couplés en parallèle, l'un pouvant pallier le défaut de fonctionnement de l'autre.

Avantageusement, l'appareil de l'invention comprend en outre un clapet anti-retour disposé dans le circuit d'amenée de l'ozone produit vers le venturi, pour empêcher la remontée d'eau vers le tube générateur.

Il peut également comprendre un détecteur de présence d'eau dans le circuit d'amenée de l'ozone produit vers le venturi, ledit détecteur étant apte à déclencher immédiatement le relais d'alimentation du transformateur HT, en cas de présence d'eau ou d'humidité.

Selon une modalité d'exécution préférée, le détecteur de présence d'eau comprend deux vis en acier inoxydable, se faisant face, et légèrement écartées.

L'appareil de l'invention peut être adapté pour être capable de fournir plusieurs valeurs de haute tension comprises entre 5 et 6kV, et par suite plusieurs concentrations différentes d'ozone dans l'eau.

Avantageusement, la concentration en ozone dissous délivré par l'appareil est comprise entre 0,03 et 1,5ppm, de préférence entre 0,03 et 0,08ppm.

### Brève description des figures

La figure 1, déjà mentionnée ci-dessus, représente le schéma des fluides dans le générateur d'ozone dissous dans l'eau selon l'invention.

La figure 2 représente schématiquement l'implantation des composants électriques dans le générateur de la figure 1.

La figure 3 montre le schéma de principe du contacteur différentiel implanté sur la carte électronique, selon la présente invention.

### Description détaillée de l'invention

Dans l'appareil générateur d'ozone dissous proposé par la présente invention, des contacteurs différentiels d'une très grande sensibilité (2mA) ont été utilisés.

Une autre caractéristique innovante de la présente invention est que ces relais de seuil sont intégrés au sein de la partie électronique du circuit imprimé.

Les détails de cet appareil sont donnés dans la description d'une forme d'exécution préférée qui suit et qui comprend notamment un circuit électronique combinant les fonctions "privé" et "professionnel" (sélection possible de trois taux d'ozone différents).

### Description d'une forme d'exécution préférée de l'invention Equipement technique

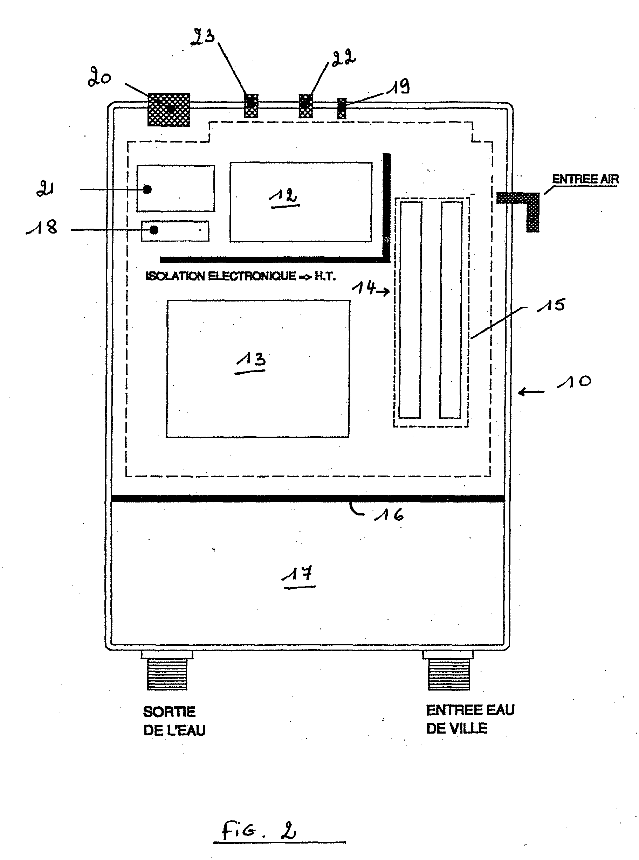

Schématiquement, comme représenté à la figure 2, et selon une modalité préférée d'exécution de l'invention, le groupe générateur est composé de deux sous-ensembles électriques, à savoir :
- un transformateur d'isolement 220/12VAC - 60VA, situé en dehors du boîtier générateur proprement dit, pour alimenter l'ensemble sous très basse tension (non représenté) ;
- un boîtier 10 comprenant un circuit de contrôle et de commande 12, ce dernier étant complètement noyé dans la résine, un transformateur 13 haute tension (HT) 12/6000VAC pour alimenter les tubes générateurs d'ozone 14.

Côté fluides, l'ozone 8 produit dans les tubes 14 à blindage EMC 15 passe dans un concentrateur d'ozone 7 placé dans un flexible de sortie, un détecteur de présence d'eau 6, un clapet anti-retour 5 et arrive au venturi 3. Dans le venturi 3, l'ozone est mélangé à de l'eau courante 1 qui y est acheminée, via un débitmètre à palettes 2 (mécanique ou électronique à impulsions), sous une pression variant de 3 à 7 bars. L'eau chargée d'ozone est ensuite disponible pour le puisage 4 (voir figure 1). Le circuit spécifique 12 assure que le mélange d'eau et d'ozone, produit directement à la sortie de l'appareil, est maintenu constant et homogène. Le circuit 12 permet aussi de maintenir le débit d'eau constant.

Le boîtier 10 est compartimenté (cloison 16) de manière à séparer l'électronique de la zone de contrôle débit/mélange de l'ozone à l'eau 17 (partie hydraulique).

Enfin, il convient de prévoir un étiquetage légalisé et informatif sur les différentes tensions de service et les dangers d'électrocution.

### Principe de fonctionnement

Le boîtier principal est raccordé au réseau d'eau de ville. A l'intérieur du coffret, le débitmètre à palettes 2 génère des impulsions lors d'un puisage, qui sont transmises à la carte électronique 12, ce qui a pour effet d'enclencher les relais 18 de commande auxiliaire et de puissance du transformateur HT. La génération d'ozone démarre à ce moment.

L'ozone produit dans les tubes 14 montés en parallèle est amené au venturi 3 par des flexibles en plastique, en passant au préalable dans le clapet anti-retour 5, qui constitue le premier point de sécurité.

L'élément de contrôle de présence d'eau 6 a pour but de déclencher les relais d'alimentation HT en cas de mauvais fonctionnement du clapet anti-retour, ce qui constitue le deuxième point de sécurité. Le fonctionnement du système est donc complètement bloqué en cas de détection de remontée d'eau vers les tubes générateurs d'ozone.

Un troisième point de sécurité consiste dans le contrôle d'un éventuel courant de fuite, côté HT, selon le principe du disjoncteur différentiel (relais de seuil), dont la cause peut être une manipulation accidentelle, un dysfonctionnement du clapet anti-retour ou encore la remontée d'eau éventuellement non détectée vers les tubes. Cette troisième sécurité permet de couper immédiatement l'alimentation HT dans le cas de défaillance simultanée du clapet anti-retour du venturi et du détecteur d'eau dans le conduit amenant l'ozone vers le venturi, en vue de la protection vitale d'une personne au travers de laquelle il pourrait y avoir effet de retour de la haute tension vers la terre, en cas de défaillance du système.

On notera que tout le système est piloté par un circuit microcontrôleur, à l'exception de la "détection différentielle" qui, encore par précaution, possède son propre circuit individuel.

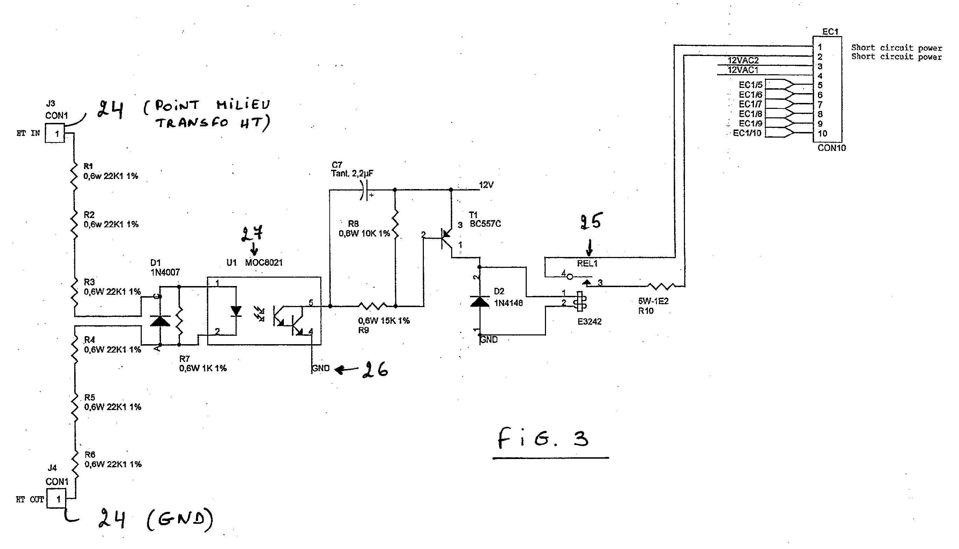

Avant d'être relié à la terre 26, le point milieu du secondaire du transformateur HT (6kV) est connecté à un réseau de résistances limitatrices R1, ..., R6 et un opto-coupleur 27, tous deux situés sur le circuit imprimé (figure 3). Dès qu'il y a détection du passage d'un courant seuil de 2mA, un relais 25 entre en action et court-circuite le fusible de protection 22 de la carte électronique (200mA rapide). Par précaution encore, ce relais 25 est commandé par deux transistors, couplés en parallèle, l'un pouvant palier un défaut de fonctionnement de l'autre. La carte n'étant plus alimentée, les relais de commande du transformateur HT sont déclenchés, ce qui coupe l'alimentation HT.

En plus de ces trois sécurités, il en existe une quatrième, qui consiste à empêcher le fonctionnement de la carte électronique, par l'intermédiaire d'un interrupteur situé à l'arrière du boîtier, côté extérieur, pour le cas où ce dernier n'est pas correctement fixé sur un support adéquat (en principe, vertical).

Le couvercle du boîtier comporte encore une diode LED de signalisation 19 (vert : production d'ozone, rouge : défaut de fonctionnement / présence d'eau).

On trouve enfin un interrupteur principal 20 avec protection par fusible 23 (5A lent) en plus d'un filtre réseau "Schaffner" 21.

La plaque d'embase, ainsi que les composants implantés, vernis, etc., sont soigneusement choisis, selon les normes ou les règles bien connues de l'homme de métier, de manière à résister aux hautes températures et surtout, à assurer l'isolement indispensable.

Le contrôleur électronique de l'appareil prend également en compte différents paramètres influençant la production d'ozone, tels que la dureté, la pression et la température de l'eau utilisée.

### Propriétés fonctionnelles spéciales.

Comme représenté sur le schéma à la figure 3, la carte électronique 12 est équipée d'un système de fusion de fusible d'alimentation de la carte faisant appel à un différentiel à très haute sensibilité (2mA - temps de réaction < 20ms). Le différentiel est intégré sur la carte 12 pour plus de sécurité. Il est ainsi raccordé entre le point milieu de la sortie HT du transformateur qui alimente les tubes générateurs et la terre 26, via des colonnettes hexagonales 24 implantées à même le circuit imprimé de contrôle 12. L'optocoupleur U1 (MOC8021) est du type à sortie "Darlington", avec un coefficient de transfert de courant de 1000% et une tension d'isolement de 7,5kV. En cas de détection d'un courant de fuite d'au moins 2mA, l'optocoupleur 27 conduit et le relais 25 est enclenché, court-circuitant le fusible de protection de 200mA. Le but essentiel du différentiel est de protéger l'utilisateur contre tout contact accidentel avec la HT, dans le cas où un retour d'eau vers les générateurs se produirait.

Une forme d'exécution pour la détection de remontée d'eau dans le tube qui conduit l'ozone vers le venturi (Figure 1, repère 6) est constituée de deux vis en acier inoxydable, se faisant face, et légèrement écartées. En cas de présence d'eau ou d'humidité, ce système déclenche immédiatement le relais d'alimentation du transformateur HT et sa réaction est signalée par le clignotement rapide de la diode LED de signalisation 19 (rouge).

Ainsi, la mise sous tension du transformateur HT ne peut se faire qu'à trois conditions remplies simultanément :
- pas de détection de remontée d'eau ;
- présence d'un débit d'eau, sous contrôle du débitmètre relié au microcontrôleur muni d'une routine "watchdog" (par exemple PIC16F84A) ;
- sélection du régime d'ozone (par exemple 3 régimes disponibles).

Le modèle "professionnel" dispose de la possibilité de sélection de trois taux d'ozone différents, correspondant à trois valeurs respectives de HT (5, 5,5 et 6kV), suivant le relais enclenché qui alimente un des trois circuits primaires du transformateur HT. La sélection s'effectue au moyen d'un bouton-poussoir, installé sur une des faces du boîtier principal. Ce bouton-poussoir envoie un signal vers le microcontrôleur qui transite par la carte d'affichage.

## Revendications

1. Appareil électronique doseur d'ozone dissous dans l'eau, comprenant au moins un tube générateur (14) de l'ozone à partir d'oxygène diatomique pur ou présent dans un gaz et un venturi (3) connecté d'une part audit générateur (14) et d'autre part à une alimentation en eau (1), ledit venturi servant au mélange de l'ozone produit avec l'eau, ainsi qu'un boîtier électrique (10) contenant un transformateur (13) pour l'alimentation haute tension (HT) du tube générateur et une carte électronique de commande et de contrôle à microcontrôleur (12), **caractérisé en ce que** ledit appareil comprend des moyens de sécurité pour détecter un courant de fuite au niveau de la haute tension, notamment en cas de remontée d'eau ou de présence d'humidité au niveau du tube générateur.

2. Appareil selon la revendication 1, **caractérisé en ce que** lesdits moyens comprennent un contacteur différentiel ultrasensible permettant de court-circuiter un fusible rapide de protection (22) de la carte électronique (12), ce qui coupe l'alimentation HT, par détection du passage d'un courant de fuite à partir d'un seuil d'au plus 2mA en un temps inférieur à 20ms.

3. Appareil selon la revendication 2, **caractérisé en ce** le contacteur différentiel est tel que le point milieu du transformateur HT est connecté à un réseau de résistances limitatives de tension (R1, ..., R6) et est relié à la terre (26) via un optocoupleur à sortie Darlington (27), qui commande, via un transistor, un relais (25) mettant en court-circuit ledit fusible rapide de protection (22).

4. Appareil selon la revendication 2 ou 3, **caractérisé en ce que** le contacteur différentiel est intégré à la carte électronique (12) et **en ce que** la haute tension du transformateur (13) d'alimentation du tube générateur d'ozone est amenée sur la carte (12) au moyen de colonnettes hexagonales (24) fixées à la carte et de hauteur minimale 20mm.

5. Appareil selon la revendication 3, **caractérisé en ce que** le relais (25) est commandé par deux transistors couplés en parallèle, l'un pouvant pallier le défaut de fonctionnement de l'autre.

6. Appareil selon l'une quelconque des revendications précédentes, **caractérisé en ce qu'**il comprend en outre un clapet anti-retour (5) disposé dans le circuit d'amenée de l'ozone produit vers le venturi (3), pour empêcher la remontée d'eau vers le tube générateur (14).

7. Appareil selon la revendication 6, **caractérisé en ce qu'**il comprend un détecteur de présence d'eau (6) dans le circuit d'amenée de l'ozone produit vers le venturi (3), ledit détecteur étant apte à déclencher immédiatement le relais d'alimentation du transformateur HT, en cas de présence d'eau ou d'humidité.

8. Appareil selon la revendication 7, **caractérisé en ce que** le détecteur de présence d'eau (6) comprend deux vis en acier inoxydable, se faisant face, et légèrement écartées.

9. Appareil selon l'une quelconque des revendications précédentes, **caractérisé en ce qu'**il est capable de fournir plusieurs valeurs de haute tension comprises entre 5 et 6kV.

10. Appareil selon l'une quelconque des revendications précédentes, **caractérisé en ce que** la concentration en ozone dissous délivré par l'appareil est comprise entre 0,03 et 1,5ppm, de préférence entre 0,03 et 0,08ppm.
